# EUROPEAN PATENT APPLICATION

(11) **EP 0 648 497 A1**
(43) Date of publication of application: **19.04.1995**
(21) Application number: 93913558.8
(22) Date of filing: 22.06.1993
(51) Int. Cl.: A61K 38/00, A61K 9/107

(54) **EMULSIFIED PREPARATION OF AUREOBASIDIN**

(30) Priority: 26.06.1992 JP 191347/92; 15.04.1993 JP 111133/93
(71) Applicant: NIPPON KAYAKU KABUSHIKI KAISHA, Tokyo 102 (JP)
(72) Inventor: OHTSUKI, Kazuo, Kita-ku, Tokyo 115 (JP); OHKUMA, Takaaki, Kitaadachi-gun, Saitama 369-01 (JP); TAKASHIO, Kazutoshi, Takasaki-shi, Gunma 370-12 (JP); TAKAHASHI, Wataru, Takasaki-shi, Gunma 370-12 (JP)
(74) Representative: Türk, Dietmar, Dr. rer. nat.
(86) International application number: JP9300840
(87) International publication number: WO9400142

(57) **Abstract**

An object of the invention is to make aureobasidins, useful as an antifungal agent, usable in the form of an emulsified preparation for, for example, injection. This preparation contains an aureobasidin, fat or oil, and water as the essential ingredients, and an oily phase containing the aureobasidin is dispersed in a water phase to form an emulsion. A preferable concentration of the fat or oil ranges from 1 to 30 % w/v. The preparation further contains an emulsifier comprising a phospholipid and/or polyoxyethylene-polyoxypropylene glycol and optionally a dissolution aid comprising an aromatic carboxylic acid and an ester and an emulsion stabilizer comprising a higher fatty acid or an acidic phospholipid.

## Description

### TECHNICAL FIELD

The present invention relates to an emulsion preparation of aureobasidins useful as an antifungal agent. The preparation can be used, for example, as an injection preparation and the like.

### BACKGROUND ART

Aureobasidins are known compounds and are produced, for example, by a strain which belongs to Genus Aureobasidium (International Deposit No. FERM-BP1938). They are expectedly useful as antifungal agents (JP-A-Nos. 2-138296, 3-22995 and 3-44398). Mycosis profunda is a fatal disease to such patients under immunosuppressive conditions as are suffering from cancer or acquired immunodeficiency syndrome and the like or have undergone organ transplantation, and has been increasing in recent years. For these patients under grave conditions, a preparation of aureobasidins which can be administered by injection is eagerly awaited.

Aureobasidins are cyclic peptides substantially insoluble in water and can difficultly be made up into an injection preparation by dissolution in water. A generally used method for making up a water-insoluble medicine into an injection preparation is micellization by using a nonionic surface active agent. For example, it is widely practiced to form injection preparations of fat-soluble vitamins by solubilizing them in water with the aid of surface active agents. As an example of antifungal agents, a preparation of miconazole solubilized with the aid of a surface active agent is available on the market as an injection preparation. As to cyclosporin A, which is a cyclic peptide sparingly soluble in water, also an injection preparation is prepared by solubilizing it with a surface active agent and is on the market. Surface active agents generally used for making injection preparations include nonionic surface active agents which exhibit low kemolytic effect and low tissue injuring effect, polyoxyethylene castor oil derivatives, polyoxyethylene hydrogenated castor oil derivatives, polyoxyethylene sorbitan fatty acid esters, and polyoxyethylene polyoxypropylene glycols. Among these active agents, polyoxyethylene castor oil derivatives and polyoxyethylene hydrogenated castor oil derivatives are known to cause anaphylactic shocks on rare occasions when injected in some of the patients and hence their use is not preferable. Further, aureobasidins are very difficult to solubilize through micelle formation, and cannot be solubilized by use of polyoxyethylene sorbitan fatty acid esters or polyoxyethylene polyoxypropylene glycols.

Accordingly, the development of a pharmaceutical preparation which is highly safe and can be used for injection is eagerly desired.

### DISCLOSURE OF THE INVENTION

The present inventors have made extensive study to make up aureobasidins, which are substantially insoluble in water, into a preparation which can be used also for injection and has a high safety. As the result, it has been found that a preparation which has a good stability and a high safety can be obtained when aureobasidins are made up into an emulsion preparation comprising aureobasidins, a fatty oil, and water as essential ingredients and obtainable by dispersing and emulsifying an oil phase containing aureobasidins in an aqueous phase. Thus, the present invention has been accomplished.

The emulsion preparation of the present invention is usually obtained by dissolving an aureobasidin in a fatty oil, then adding an emulsifying agent to the solution to form a uniformly dispersed suspension and then adding an appropriate amount of water and emulsifying the mixture.

### BRIEF DESCRIPTION OF THE DRAWINGS

Fig. 1 is a graph showing survival rate curves obtained when the preparation of Example 1 of the present invention and a control preparation are administered to Candida-infected mice.

Fig. 2 is a graph showing survival rate curves obtained when the preparation of Example 2 of the present invention and a control preparation are administered to Candida-infected mice.

Fig. 3 is a graph showing body weight change curves obtained in the cases where the preparation of Example 1 of the present invention and a control preparation are administered to Candida-infected mice and in the case of a non-infected, untreated mice.

Fig. 4 is a graph showing body weight change curves obtained in the cases where the preparation of Example 2 of the present invention and a control preparation are administered to Candida-infected mice and in the case of a non-infected, untreated nice.

### BEST MODE FOR CARRYING OUT THE INVENTION

The aureobasidins used in the present invention may be the novel antibiotic substance R106 (aureobasidin A, hereinafter abbreviated as R106-I), its analogous compounds and its derivatives described in JP-A-No. 2-138296 (USA-5057493, USA-5158876, EPA-352092). Specifically, they include the compounds disclosed in the above patent publications and the descriptions of these publications are incorporated by reference.

Fatty oils which can be used for dissolving aureobasidins include vegetable oils of natural origin, such as soybean oil, safflower oil, corn oil, olive oil, sesame oil, peanut oil, cotton seed oil, rape seed oil and coconut oil, and synthetic fatty oils and the like such as fatty acid triglycerides (the fatty acid having 6-22, preferably 8-20, carbon atoms). From the viewpoint of solubility, the use of medium chain (having 8-12 carbon atoms) triglycerides, e.g., capric acid triglyceride and caprylic acid triglyceride, is preferable. Further, two or more of these fatty oils may be used in admixture for improving the solubility of aureobasidins in fatty oils and for like purposes. These fatty oils are preferably liquid at ordinary temperature (about 10-30°C). Though the amount of fatty oils used varies depending on the kind thereof, it is usually 5 parts (by weight, the same applies hereinafter) or more, preferably in the range of 10 parts to 100 parts, more preferably 20 parts to 50 parts, per 1 part of aureobasidins.

The emulsifying agent used for dispersing and emulsifying an oil phase containing aureobasidins dissolved therein into an aqueous phase may be those which can be used for pharmaceutical preparations. In general, phospholipids comprising phosphatidylcholine as the main component or polyoxyethylene polyoxypropylene glycols are preferred.

The phospholipids used include phospholipids of natural origin, such as egg yolk phospholipid, soybean phospholipid, etc., which have been Purified for injection, or phospholipids obtained by hydrogenation of these, and synthetic phospholipids. The amount of phospholipids to be added is in the range of 1 part to 200 parts, preferably 5 parts to 150 parts, more preferably 10 parts to 100 parts, most preferably 10 parts to 50 parts, per 100 parts of the oil phase.

The polyoxyethylene polyoxypropylene glycols used may be obtained by addition polymerization of ethylene oxide to polypropylene glycol and is represented by the molecular formula HO(C₂H₄O)ₐ(C₃H₆O)_{b} (C₂H₄O)ₐH in which the average degree a of polymerization of ethylene oxide is preferably 12-141 and the average degree b of polymerization of propylene oxide is preferably 20-56. More preferably, the average molecular weight of the polypropylene glycol portion is about 1800 and the average molecular weight of the ethylene glycol portion is about 80% of the average molecular weight of the whole. Thus, polyoxyethylene-[160] polyoxypropylene[30] glycol (Poloxamer 188), wherein a is about 80 and b is about 30, is usually employed. The amount of these polyoxyethylene polyoxypropylene glycols to be added is in the range of 1 to 200 parts, preferably 5 parts to 150 parts, more preferably 10 parts to 100 parts, most preferably 15 parts to 50 parts, per 100 parts of the oil phase.

Further, the polyoxyethylene polyoxypropylene glycols and the phospholipids may also be used in combination. When used in combination, the amount thereof to be added is, in terms of the sum of the two, in the range of 1 part to 200 parts, preferably 5 parts to 150 parts, more preferably 10 parts to 100 parts, most preferably 15 parts to 50 parts, per 100 parts of the oil phase. The ratio of the amounts of the two to be added in combined use is about 1 part - 100 parts, preferably about 10-50 parts, of phospholipid relative to 100 parts of polyoxyethylene polyoxypropylene glycol.

Water is used in such an amount as to give a concentration of aureobasidins in the ultimate preparation of 0.01%-5% w/v, preferably 0.1-2% w/v, most generally about 0.2-1% w/v.

The emulsion preparation of the present invention may also contain other pharmaceutical additives in addition to the above-mentioned ingredients. Examples of such additives include solubilizers for aureobasidins, emulsion stabilizers, pH adjusters, buffering agents, tonicity adjusters, antioxidants, preservatives, etc.

As the solubilizers for aureobasidins, mention may be made of aromatic carboxylic acids, their esters, etc. For example, benzoic acids (benzoic acid, hydroxylbenzoic acids having 1-2 hydroxyl groups, etc.), ester derivatives of these carboxyl groups with lower alcohols (having 1-4 carbon atoms) and ester derivatives of these hydroxyl groups with lower fatty acids (having 1-3 carbon atoms) may be used. The amount of these to be added is usually 0.5 part - 20 parts, preferably 1 part - 10 parts, per 10 parts of aureobasidins.

The emulsion stabilizers are preferably medium or higher fatty acids which have the effect of preventing the flocculation and coalescence of fat emulsion particles with one another; they may be used without any particular limitation so long as they are pharmaceutically acceptable. Generally, saturated or unsaturated fatty acids having about 8-24 carbon atoms, preferably 10-20 carbon atoms, are used. Most preferred are unsaturated fatty acids having 14-20 carbon atoms. Specific examples of these fatty acids include myristic acid, palmitic acid, stearic acid, oleic acid, linolic acid, linolenic acid, arachidonic acid, etc., oleic acid being most preferred. The addition of these compounds is also preferable from the fact that they have the effect of improving the solubility of aureobasidins in fatty oils. The amount of these fatty acids added is 0.1%-40% w/w, preferably 1%-20% w/w, usually about 2% w/w-15% w/w, based on the amount of fatty oils.

Acidic phospholipids may also be used as the emulsion stabilizer, which include acidic phospholipids of natural origin and synthetic phospholipids. The acidic phospholipids may be, for example, phosphatidic acid, phosphatidyl glycerol, phosphatidyl serine, phosphatidyl inositol, etc. They are added in an amount of 0.1%-50% w/w, preferably 0.5-20% w/w, more preferably 1-10% w/w, based on the amount of the emulsifying agent.

The amount of the solubilizer and the emulsion stabilizer to be added is, in terms of the sum of the two, usually 0-20% w/v, preferably 0.1-10% w/v, relative to the whole of the emulsion preparation.

The pH adjusters and the buffering agents used may be physiologically highly safe acids and bases conventionally used for injections. Examples thereof include inorganic acids such as hydrochloric acid, sulfuric acid, phosphoric acid, etc., organic acids such as citric acid, etc., and alkaline metal salts thereof. The pH to be adjusted is not particularly limited so long as it is physiologically acceptable, but it is preferably in the range of 5-9, generally 6-8, in order to keep safety and stability of emulsion.

The tonicity adjusters may be salts, sugars, sugar alcohols and alcohols which have a high physiological safety. To keep the stability of the emulsion preparation, the use of nonionic substances, for example, sugars such as glucose, etc., sugar alcohols such as mannitol, etc., polyhydric alcohols such as glycerol, etc., and C₂-C₄ lower alcohols and the like is preferable. They are added in an amount necessary for maintaining the tonicity of the emulsion preparation at a physiologically safe level. They are usually added in an amount necessary for giving a ratio of the tonicity of the preparation to that of a isotonic sodium chloride solution of 0.7 or more, preferably 0.7-4.

As to other additives, mention may be made of antioxidants, preservatives, and the like. Particularly when the preparation contains fatty oils containing unsaturated bonds, fat-soluble antioxidants, such as vitamin E, butylhydroxytoluene, butylhydroxyanisol, etc., may be used as the antioxidant to prevent the oxidation of the fatty oils. The amount of these antioxidants to be added is usually in a range of amount from 0.0001 part to 0.01 part per 1 part of fatty oil. Further, in order to prevent the degradation caused by oxidation of lipid, it is conventionally practiced to replace the atmosphere in the vessel with an inert gas, such as nitrogen gas.

Since aureobasidins are substantially insoluble in water, when the emulsion preparation is stored for a long period, the crystal of aureobasidins may tend to precipitate in the emulsion. To prevent the precipitation of the crystal, polar organic solvents having a high physiological safety conventionally used for injections, for example, ethanol, glycerol, etc. can be used. The polar organic solvent, such as ethanol, is added in an amount of 20% w/v or less, preferably 1-10% w/v, relative to the emulsion preparation.

The process for producing the emulsion preparation of the present invention is described in more detail below.

The dissolution of aureobasidins into fatty oils may be carried out at ordinary temperature but is generally preferably dissolved with heating at about 30-80°C. When a solubilizer, emulsion stabilizer, preservative, etc. are used, they are preferably mixed and dissolved into the fatty oil beforehand, but they may also be added later as occasion demands.

Usually an emulsifying agent is added to a fatty oil solution containing aureobasidins dissolved therein, and the resultant mixture is thoroughly mixed to form a uniform dispersion. The mixing of the emulsifying agent may be conducted either under heating or at ordinary temperature.

The emulsification step can be conducted by adding a fatty oil solution containing an emulsifying agent dispersed therein to an aqueous phase and then emulsifying the mixture by using an emulsifier. The emulsifier used may be such an emulsifier as homomixer and homogenizer. For producing an emulsion preparation having a favorable particle diameter for use as injections, a high pressure emulsifying apparatus as a Manton-Gaulin (a registered trade mark) type homogenizer and a Microfluidizer (a registered trade mark) is preferably used for emulsification.

To ensure the stability of the emulsion preparation and the safety thereof when injected, the particle diameters of emulsified fatty oil are preferably in the range of 0.05-1 µm and the mean particle diameter is preferably 0.4 µm or less.

The emulsion thus obtained may be treated with a membrane filter to remove foreign substances. The filtered emulsion may also be filled in a sterilized vessel, hermetically sealed and then sterilized by autoclave.

Preferred compositions of the emulsion preparation thus obtained are shown below. The proportions of respective ingredients are all relative to the whole of the emulsion preparation.

| | |
|---|---|
| Aureobasidin | 0.01-5% w/v, preferably 0.1-2% w/v |
| Fatty oil | 1-30% w/v, preferably 5-20% w/v |
| Emulsifying agent | 0.5-20% w/v, preferably 1-10% w/v |
| Other pharmaceutical additives | 0-20% w/v, preferably 1-10% w/v |
| Water | the balance |

More preferred compositions are shown below. All of the proportions are based on the whole of the emulsion preparation.

| | |
|---|---|
| Aureobasidin R106-I | 0.01-5% w/v, preferably 0.1-2% w/v |
| C₈-C₂₀ triglyceride (including vegetable oils of natural origin) as fatty oil | 1-30% w/v, preferably 5-20% w/v |
| Polyoxyethylene polyoxypropylene glycols, or polyoxyethylene polyoxypropylene glycol in combination with phospholipids, as emulsifying agent | 0.5-20% w/v, preferably 1-8% w/v |
| Oleic acid as emulsion stabilizer | 0.1-5% w/v, preferably 0.2-2% w/v |
| Other pharmaceutical additives | 0-18% w/v, preferably 1-8% w/v |
| Water | the balance |

### Example

The present invention is described in detail below with reference to Examples, but the invention is in no way limited to these Examples:

### Example 1

In a mixture of 9.75 g of soybean oil and 0.25 g of oleic acid was dissolved 0.25 g of aureobasidin R106-I with heating at 50-60°C, and then 1.2 g of purified egg yolk phospholipid was uniformly dispersed and suspended therein. To the resulting mixture were added 1.25 g of glycerol and then a suitable amount of water for injection to make up the total into 50 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The resulting emulsion was adjusted to pH 6.6 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, and then the vial was hermetically sealed. The injection preparation thus obtained was a uniform, white emulsion, which had a mean particle diameter of 0.26 µm as determined with a particle sizer by dynamic light scattering method.

### Example 2

In 10 g of soybean oil was dissolved 0.25 g of aureobasidin R106-I with heating at 50-60°C, and then 1.2 g of purified egg yolk phospholipid was uniformly dispersed and suspended therein. To the resulting mixture were added 1.25 g of glycerol and then a suitable amount of water for injection to make up the total into 50 ml, and the mixture was subjected to primary esterification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 6.5 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, and then the vial was hermetically sealed. The injection preparation thus obtained was a uniform, white emulsion, which had a mean particle diameter of 0.29 µm as determined with a particle sizer by dynamic light scattering method.

### Example 3

In a mixture of 4.75 g of caprylic acid triglyceride (Panacet 800, mfd. by Nippon Oil & Fats Co., Ltd.) and 0.25 g of oleic acid was dissolved 0.25 g of aureobasidin R106-I with heating at 50-60°C. Then 1.0 g purified egg yolk phospholipid was uniformly dispersed and suspended therein. To the resulting mixture were added 1.25 g of glycerol and then a suitable amount of water for injection to make up the total into 50 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.4 by addition of suitable amounts of hydrochloric acid and sodium hydroxide. The emulsion thus obtained was filtered through a membrane filter (pore size 0.2 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.24 µm as determined with a particle sizer by dynamic light scattering method, showing virtually no change from the mean particle diameter of 0.18 µm determined before sterilization. Further, no decrease in content by sterilization was observed, the content being 101.5% of that before sterilization.

### Example 4

In a mixture of 4.75 g of soybean oil and 0.25 g of oleic acid was dissolved 0.125 g of aureobasidin R106-I with heating at 50-60°C, and 1.0 g of hydrogenated soybean phospholipid was uniformly dispersed and suspended therein. To the resulting mixture were added 1.25 g of glycerol and then a suitable amount of water for injection to make up the total into 50 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.5 by addition of suitable amounts of hydrochloric acid and sodium hydroxide. The emulsion thus obtained was filtered through a membrane filter (pore size 0.2 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.17 µm as determined with a particle sizer by dynamic light scattering method. Further, no decrease in content by sterilization was observed, the content being 100.4% of that before sterilization.

### Example 5

In a mixture of 9.5 g of soybean oil and 0.5 g of oleic acid was dissolved 0.5 g of aureobasidin R106-I with heating at 50-60°C, and 1.2 g of purified egg yolk phospholipid was uniformly dispersed and suspended therein. To the resulting mixture were added 2.5 g of glycerol and then a suitable amount of water for injection to make up the total into 100 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.5 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.22 µm as determined with a particle sizer by dynamic light scattering method, showing virtually no change from the mean particle diameter of 0.20 µm determined before sterilization. Further, no decrease in content by sterilization was observed, the content being 97.6% of that before sterilization.

### Example 6

In a mixture of 4.75 g of soybean oil and 0.25 g of oleic acid was dissolved 0.25 g of aureobasidin R106-I with heating at 50-60°C, and 5 g of purified egg yolk phospholipid was uniformly dispersed and suspended therein. To the resulting mixture were added 2 g of glycerol and then a suitable amount of water for injection to make up the total into 100 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.3 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.11 µm as determined with a particle sizer by dynamic light scattering method, showing no change from the mean particle diameter of 0.11 µm determined before sterilization. Further, no decrease in content by sterilization was observed, the content being 103.6% of that before sterilization.

### Example 7

In a mixture of 9.5 g of soybean oil and 0.5 g of oleic acid were dissolved 0.5 g of aureobasidin R106-I and 0.5 g of propylparaben with heating at 50-60°C, and 2 g of purified egg yolk phospholipid was uniformly dispersed and suspended therein. To the resulting mixture were added 2 g of glycerol and then a suitable amount of water for injection to make up the total into 100 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.4 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.20 µm as determined with a particle sizer by dynamic light scattering method, showing no change from the mean particle diameter of 0.21 µm determined before sterilization. Further, no decrease in content by sterilization was observed, the content being 95.9% of that before sterilization.

### Example 8

In a mixture of 31.5 g of soybean oil and 3.5 g of oleic acid was dissolved 1.75 g of aureobasidin R106-I with heating at 50-60°C, and 7 g of purified egg yolk phospholipid was uniformly dispersed and suspended therein. To the resulting mixture were added a suitable amount of water for injection and 17.5 g of ethanol to make up the total into 350 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.2 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.18 µm as determined with a particle sizer by dynamic light scattering method, showing no change from the mean particle diameter of 0.18 µm determined before sterilization. Further, no decrease in content by sterilization was observed, the content being 102.3% of that before sterilization.

### Example 9

In 20 g of a medium chain triglyceride (Panacet 810, mfd. by Nippon Oil & Fats Co., Ltd.) was dissolved 0.4 g of aureobasidin R106-I with heating at 50-60°C, and 4 g of purified egg yolk phospholipid was uniformly dispersed and suspended therein. To the resulting mixture were added 4 g of glycerol and then a suitable amount of water for injection to make up the total into 200 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 7.1 by addition of suitable amounts of hydrochloric acid and sodium hydroxide. The emulsion thus obtained was filtered through a membrane filter (pore size 0.4 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.14 µm as determined with a particle sizer by dynamic light scattering method, showing no change from the mean particle diameter of 0.15 µm determined before sterilization. No decrease in content by sterilization was observed, the content being 98.0% of that before sterilization.

### Example 10

In 9 g of soybean oil and 1 g of oleic acid was dissolved 0.5 g of aureobasidin R106-I, and 2.5 g of polyoxyethylene[160]polyoxypropylene[30] glycol was uniformly dispersed and suspended therein. To the resulting mixture were added 2 g of glycerol and then a suitable amount of water for injection to make up the total into 100 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 6.6 by addition of suitable amounts of hydrochloric acid and sodium hydroxide. The emulsion thus obtained was filtered through a membrane filter (pore size 0.45 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.15 µm as determined with a particle sizer by dynamic light scattering method, showing virtually no change from the mean particle diameter of 0.16 µm determined before sterilization. No decrease in content by sterilization was observed, the content being 96.8% of that before sterilization.

### Example 11

In 1 g of ethanol was dissolved 0.5 g of aureobasidin R106-I, and then 9 g of soybean oil and 1 g of oleic acid was added thereto to form a uniform solution. Then 2.5 g of polyoxyethylene[160]polyoxypropylene[30] glycol and 0.5 g of purified egg yolk phospholipid were uniformly dispersed and suspended in the above solution. To the resulting mixture were added a suitable amount of water for injection and 4 g of ethanol to make up the total into 100 ml, and the mixture was subjected to primary emulsification by using a homogenizer (mfd. by IKA-LABORTECHNIK). The emulsion obtained was further subjected to secondary emulsification at high pressure by using a Microfluidizer (mfd. by Microfluidics Corp.). The emulsified liquid was adjusted to pH 6.6 by addition of suitable amounts of hydrochloric acid and sodium hydroxide. The emulsion thus obtained was filtered through a membrane filter (pore size 0.45 µm), filled in a sterilized vial for injections, the inner atmosphere was replaced with nitrogen gas, then the vial was hermetically sealed and sterilized by autoclave (121°C, 20 minutes) to obtain an injection preparation. The injection preparation thus obtained was a uniform, white emulsion, which had a mean particle diameter of 0.11 µm as determined with a particle sizer by dynamic light scattering method, showing no change from the mean particle diameter of 0.11 µm determined before sterilization. No decrease in content by sterilization was observed, the content being 97.0% of that before sterilization. (Test Example: in vivo antifungal effect of the preparation of the present invention)

The antifungal effects of the present preparations of Examples 1 and 2 are shown below in comparison with control preparations.

Candida strain (Candida albicans TIMM1768), 2x10⁶/0.2 ml, were intravenously administered through a mouse tail vein to cause infection. For 5 days from the day of infection, the preparations of the present invention of Examples 1 and 2 were administered at a dose of 5, 10, 20 and 50 mg/Kg in terms of the dose of R106-I. Separately, the preparations of Examples 1 and 2 from which R106-I was eliminated were administered as control preparations. A group for each dose was 10 mice, and were observed for life or death and body weight for 31 days from the day of infection of fungus. In the observation of body weights, the change of body weight in the infected groups was compared with that in the untreated group which was not infected and administered with no medicinal agent.

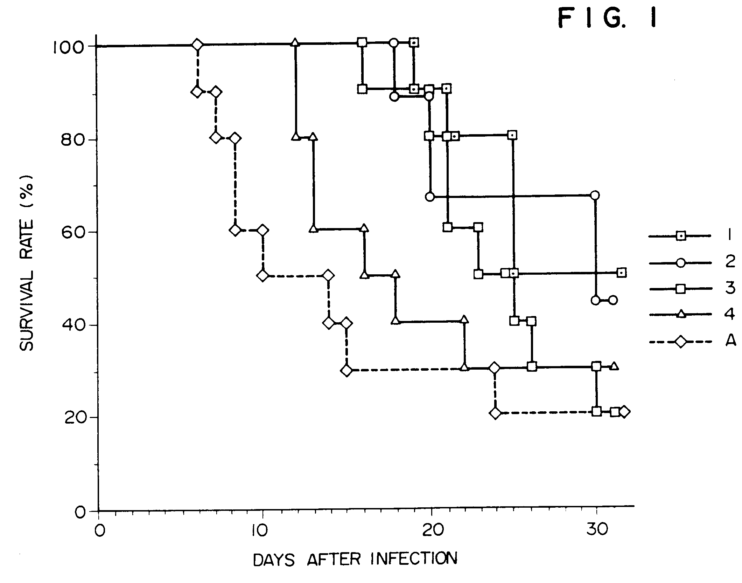

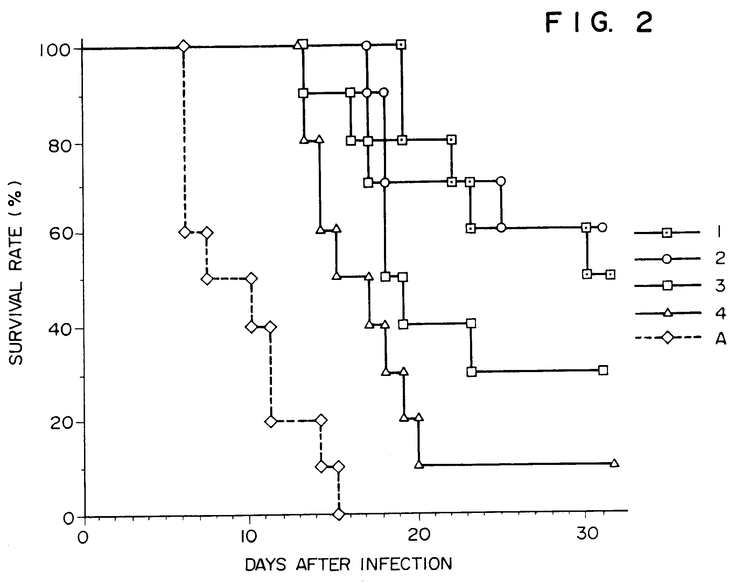

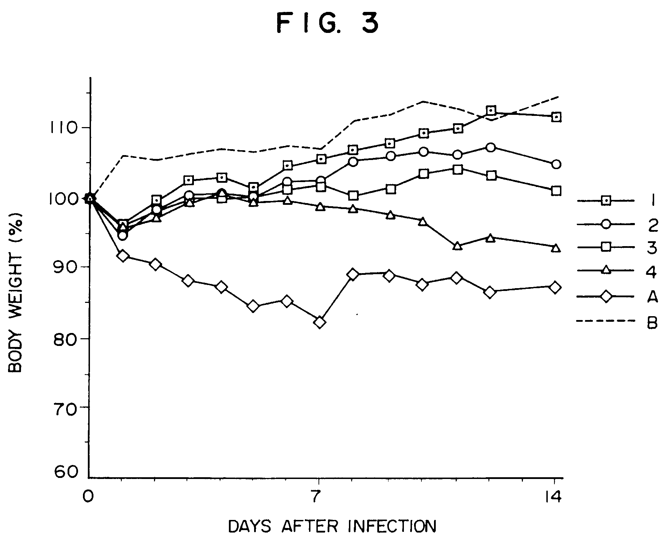

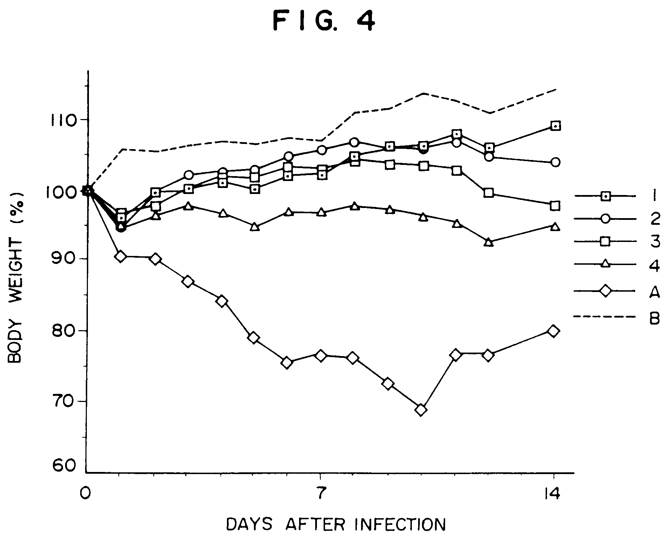

Figs. 1 and 2 respectively show the antifungal effect of the present preparations of Examples 1 and 2 by means of a survival curve in comparison with that of the control preparations. Figs. 3 and 4 respectively show the body weight change of the groups administered with the preparations of Examples 1 and 2 in comparison with that of the group administered with the control preparation and that of the untreated group.

In Fig. 1, numerals 1-4 indicate the groups to which the preparation of Example 1 was administered and A indicates the group to which the control preparation was administered. The doses of R106-I are 50 mg/kg for 1, 20 mg/kg for 2, 10 mg/kg for 3, 5 mg/kg for 4 and 0 mg/kg for A.

In Fig. 2, numerals 1-4 indicate the groups to which the preparation of Example 2 was administered and A indicates the group to which the control preparation was administered. The doses of R106-I are 50 mg/kg for 1, 20 mg/kg for 2, 10 mg/kg for 3, 5 mg/kg for 4 and 0 mg/kg for A.

In Fig. 3, numerals 1-4 indicate the groups to which the preparation of Example 1 was administered, A indicates the group to which the control preparation was administered and B indicates the non-infected untreated group. The doses of R106-I are 50 mg/kg for 1, 20 mg/kg for 2, 10 mg/kg for 3, 5 mg/kg for 4, and 0 mg/kg for A and B.

In Fig. 4, numerals 1-4 indicate the groups to which the preparation of Example 2 was administered, A indicates the group to which the control preparation was administered, and B indicates the non-infected untreated group. The doses of R106-I are 50 mg/kg for 1, 20 mg/kg for 2, 10 mg/kg for 3, 5 mg/kg for 4 and 0 mg/kg for A and B.

The preparations of the present invention show, in each of the Examples, marked increase in survival rate and recovery in body weight change with the increase of dosage. This demonstrates the excellent antifungal effect and high safety of the preparation of the present invention.

### INDUSTRIAL APPLICABILITY

The injection preparation of the present invention obtained by using a phospholipid and/or polyoxyethylene polyoxypropylene glycol as an emulsifying agent has an excellent antifungal effect, and further has no side effect such as anayhylactic shock and hence has a high safety. Further, the emulsion preparation of the present invention does not show separation of oil phase and increase of dispersed oil particle diameter in autoclave, so that it can be sterilized by autoclave. Accordingly, asepsis in preparing injections can be guaranteed without difficulty, and injection preparations having excellent stability can be obtained.

## Claims

1. An emulsion preparation characterized by comprising an aureobasidin, a fatty oil, an emulsifying agent and water as essential ingredients wherein an oil phase containing the aureobasidin is dispersed and emulsified in an aqueous phase.

2. The preparation according to claim 1 wherein the concentration of the fatty oil in the emulsion preparation is 1-30% w/v.

3. The preparation according to claim 1 which contains at least one compound selected from aromatic carboxylic acids and their esters as a solbilizer for the aureobasidin in the fatty oil.

4. The preparation according to claim 1 wherein the emulsifying agent is at least one member selected from the group consisting of phospholipids and polyoxyethylene polyoxypropylene glycols and the amount of the emulsifying agent is 1-200 parts per 100 parts of the fatty oil in the emulsion preparation.

5. The preparation according to claim 1 which contains a higher fatty acid or an acidic phospholipid.

6. The preparation according to claim 1 which contains a polar organic solvent.

7. The preparation according to claim 1 wherein the proportions of respective ingredients to the whole of the preparation are 0.1-2% w/v for the aureobasidin, 10-20% w/v for the fatty oil, 1-10% w/v for the emulsifying agent, 1-10% w/v for other pharmaceutical additives, and the remainder for water.

8. The preparation according to claim 1 wherein the emulsion stabilizer is oleic acid, the emulsifying agent is a polyoxyethylene polyoxypropylene glycol or a combination of a polyoxyethylene polyoxypropylene glycol with a phospholipid, and the amount of the emulsifying agent added is 1-5 parts per 1 part of oleic acid added.

## Amended claims

### Amended claims under Art. 19.1 PCT

1. An emulsion preparation characterized by comprising an aureobasidin, a fatty oil, an emulsifying agent and water as essential ingredients wherein an oil phase containing the aureobasidin is dispersed and emulsified in an aqueous phase.

2. The preparation according to claim 1 wherein the concentration of the fatty oil in the emulsion preparation is 1-30% w/v.

3. The preparation according to claim 1 which contains at least one compound selected from aromatic carboxylic acids and their esters as a solubilizer for the aureobasidin in the fatty oil.

4. The preparation according to claim 1 wherein the emulsifying agent is at least one member selected from the group consisting of phospholipids and polyoxyethylene polyoxypropylene glycols and the amount of the emulsifying agent is 1-200 parts per 100 parts of the fatty oil in the emulsion preparation.

5. The preparation according to claim 1 which contains a higher fatty acid or an acidic phospholipid.

6. (addition) The preparation according to claim 1 which contains a higher fatty acid in an amount of 1%-20% w/w relative to the amount of the fatty oil in the emulsion preparation or an acidic phospholipid in an amount of 0.5-20% w/w relative to the amount of the emulsion preparation.

7. (previous claim 6) The preparation according to claim 1 which contains a polar organic solvent.

8. (previous claim 7) The preparation according to claim 1 wherein the proportions of respective ingredients to the whole of the preparation are 0.1-2% w/v for the aureobasidin, 10-20% w/v for the fatty oil, 1-10% w/v for the emulsifying agent, 1-10% w/v for other pharmaceutical additives, and the remainder for water.

9. (previous claim 8) The preparation according to claim 1 wherein the emulsion stabilizer is oleic acid, the emulsifying agent is a polyoxyethylene polyoxypropylene glycol or a combination of polyoxyethylene polyoxypropylene glycol with a phospholipid, and the amount of the emulsifying agent added is 1-5 parts per 1 part of oleic acid added.
